# EUROPEAN PATENT APPLICATION

(11) **EP 3 117 870 A1**
(43) Date of publication of application: **18.01.2017**
(21) Application number: 16179425.0
(22) Date of filing: 28.09.2011
(51) Int. Cl.: A61N 1/32, G01N 33/53, C12N 5/00, B82Y 5/00

(54) **MOLECULAR DELIVERY WITH NANOWIRES**

(30) Priority: 29.09.2010 US 387604 P; 14.03.2011 US 201161452283 P
(62) Divisional of application: 11770260.5
(71) Applicant: President and Fellows of Harvard College, Cambridge, MA 02138 (US)
(72) Inventor: Park, Hongkun, Cambridge, MA 02138 (US); Robinson, Jacob, Cambridge, MA 02138 (US); Sutton, Amy, Cambridge, MA 02138 (US); Jorgolli, Marsela, Cambridge, MA 02138 (US); Shalek, Alexander K., Cambridge, MA 02138 (US)
(74) Representative: Hoffmann Eitle

(57) **Abstract**

The present invention provides a molecular delivery device including a plurality of nanowires (e.g., Si NWs) coated with an electrically conductive layer formed of a metal oxide.

## Description

### BACKGROUND

Delivery of exogenous genetic materials into cells can be achieved virally (e.g., using an adeno-associated or a lenti-viral vector), chemically (e.g., using calcium phosphate, liposome, or polycation), mechanically (e.g., microinjection), and or physically (e.g., electroporation).

Among these deferent delivery methods, electroporation is achieved by placing cells in a uniform electric field formed between two parallel electrodes. When the transmembrane potential exceeds a threshold level around 0.25 to 1 V, the lipid bilayer of cell membrane rearranges to form hydrophilic pores (typically between 20-120 nm in diameter). Any molecule smaller than the pore size, can flow into the cell either by electrophoresis or diffusion. Typically, for electroporating cells suspended in a solution, pulses of around 1000 V (varying with cell size) lasting a few microseconds to a millisecond is required.

While this method works effectively for certain cells, electroporation of small immune cells and neurons is less successful. When a uniform electric field is applied to small cells or neurons for electroporation, the potential over a large fraction of the cellular membrane is above the breakdown potential, resulting in low cell viability. There is a need to develop an improved electroporating method for these cells.

### SUMMARY

This invention is based on an unexpected discovery that a voltage waveform of less than 10 V in amplitude can efficiently electroporate a cell using a set of electrically conductive nanowires collectively as an intracellular electrode.

In one aspect, this invention features a molecular delivery device including (i) a substrate and (ii) a plurality of nanowires attached to a surface of the surface. The substrate is electrically conductive, and the nanowires are coated with an electrically conductive layer.

The electrically conductive substrate used in the above-described device can be a substrate made of a non-conductive or semiconductive material having a surface coated with an electrically conductive material and the surface is electrically communicative with the nanowires. Alternatively, it can be made of an electrically conductive material. Examples of a material for the substrate include a semiconductor (e.g., Si and Ge), a compound semiconductor (e.g., InP and GaAs), a metal oxide (e.g., ZnO, ITO, and Ir oxide), and a metal (e.g., Au, Pt, Ag, Ir, and Cr).

The term "nanowire" (or "NW" hereafter) refers to a material in the shape of a wire, rod, or cone having a diameter in the range of 1 nm to 1 µm. Herein, the cone has a half angle in the range of 0-90 degree (e.g., 0-15 degree). The NWs are preferably attached to the surface along a substantially vertical direction (i.e., 60-90 degree) to the surface. They can have a height of 20-10,000 nm (e.g., 100-5,000 nm and 800-1,200 nm), a diameter of 10-500 nm (e.g., 50-250 nm and 70-180 nm), and a density of 0.05-10 wires µm⁻² (e.g., 0.1-5 wires µm⁻² and 0.2-2 wires µm⁻²). They can be formed of a semiconductor (e.g., Si and Ge), a compound semiconductor (e.g., GaAs and InP), a metal oxide (e.g., ZnO), a metal (e.g., Au, Ag, Ir, Pt), carbon, boron nitride, or a combination thereof.

The conductive layer coated on the NWs and the substrate can be formed of a metal (e.g., Au, Ag, Pt, Pd, Cr, Ni, Ir, Al, W, Ti, and Fe), a metal oxide (e.g., Ir oxide, ITO, and ZnO), a semiconductor (e.g., Si and Ge), a compound semicoductor (e.g., GaAs, GaP, InP, InAs, InGaAs, and GaN), a metal nitride (e.g., TiN, ZrN, and TaN), or a combination thereof.

Compound semiconductor can be formed of two or more elements, such as a IV-IV semiconductor (e.g., SiC and SiGe), a III-V semiconductor (e.g., AlN, AlP, AlGaAs, GaN, GaAs, InP, and InGaAs), a II-V semiconductor (e.g., Zn₃Sb₂ and Cd₃As₂), a II-VI semiconductor (e.g., CdS, CdSe, and CdTe), a IV-VI semiconductor (e.g., SnS and PbSnTe), a I-VI semiconductor (e.g., Cu₂S), a I-VII semiconductor (e.g., CuCl), and an oxide semiconductor (e.g., SnO₂, CuO, and Cu₂O). Unless stated otherwise, the semiconductor used for the electrical device of this invention includes both its intrinsic form (i.e., pure form) and doped form (i.e., containing one or more dopants). The term "combination" refers to a mixture, an alloy, or a suitable reaction product of two or more components. For example, "a combination of silicon and a metal" can be a mixture of silicon and the metal or a silicide of the metal.

In another aspect, this invention relates to a method of delivering an exogenous molecule into a cell. The method includes providing (i) a substrate having a surface and a plurality of NWs attached to the surface, the substrate and each of the NWs being electrically conductive; (ii) contacting the NWs with a cell to allow penetration of the NWs into the cell; and (iii) applying a current or voltage waveform between the substrate and an electrode in the bath solution. As a result, the molecule enters into the cell through transiently formed pores in cell membranes. Electrically conductive NWs can be (1) made of a non-conductive or semiconductive material having a surface coated with an electrically conductive material and the surface is electrically communicative with the surface of the substrate, to which NWs are attached; or (2) made of an electrically conductive material.

The device used in this method is the same as the one described above except that the NWs used in this method can be coated or not coated with an electrically conductive layer.

The molecule to be delivered can be a nucleic acid (e.g., DNA and RNA including siRNA and microRNA), a protein, a polysaccharide, or a small molecule. The term "small molecule" refers to any molecule with a molecular weight below 1000 Da, including various drug molecules, fluorescent dyes, oligosaccharides, oligonucleotides, and peptides.

The cell can be a prokaryotic cell (e.g., E. coli) or a eukaryotic cell (e.g., a yeast cell and an human cell). The human cell can be a primary cell, a transformed cell (e.g., an HEK cell), or a cancerous cell (e.g., a HeLa cell). The primary cell can be an oocyte, a neuron, a neuroblast, a beta cell, a myocyte, an osteoblast, a fibroblast, a kerotinocyte, a monocyte, an immune cell, or a stem cell. The immune cell can be a macrophage, a natural killer cell, a T cell, and a B cell, and a dendritic cell. The stem cell can be an embryonic stem cell or an adult stem cell (e.g., hematopoietic stem cell and a mesenchymal stem cell). Preferably, each biological cell is penetrated by two or more NWs.

The electrical signal can either be an electrical current or voltage signal. The amplitude of the voltage waveform is 0.1-10 V (e.g., 3-7 V and 4-6 V). The term "waveform" is a plot of a voltage (or current) amplitude as a function of time. It is a general term for a pulse in a square, triangular, sawtooth, or sinusoidal shape.

Still another aspect of the invention relates to a method of delivering an exogenous molecule into a cell. The method includes (i) providing a substrate having a surface, which is coated with an electrically insulted layer, and a plurality of electrically conductive nanowires, each of which, having a first end and a second end, is coated with an electrically insulating layer except for the first and second ends, the first end being attached to the surface and the second end being coated with an electrically conductive layer; (ii) contacting the nanowires with the cell immersed in a bath solution containing the molecule to allow penetration of one or more nanowires into the cell; and (iii) applying a current or voltage waveform between two electrodes, one connected to the first end of each of the nanowires and the other placed in the bath solution. As a result, the molecule enters into the cell through transiently formed pores on cell membranes.

The substrate used in this method can be formed of a semiconductor (e.g., Si), a compound semiconductor (e.g., GaAs, InP, GaN, and GaP), or diamond. It is coated with an electrically insulated layer. The NWs used in this method are the same as those described above except that the region between the two ends of each of the NWs are coated with an electrically insulated layer. In addition, each NW can be individually addressable by a voltage waveform. The electrically insulating layer is formed of an oxide (e.g., silica, alumina, and hafnium oxide), a nitride (e.g., silicon nitride), or a combination thereof. Alternatively, the electrically insulating layer is formed of an organic material, such as Parylene (e.g., Parylene C, N, AF-4, SF, HT, A, AM, VT-4, or CF), polydimethylsiloxane, methyl methacrylate, a photoresist (e.g., SU-8), and an electron beam resist (e.g., polymethylmethacrylate, ZEP-520, and hydrogen silsesquioxane).

The cell can be a prokaryotic cell and a eukaryotic cell as mentioned above. The molecule to be delivered can also be a nucleic acid, a protein, a polysaccharide, or a small molecule. The electrical signal can either be an electrical current or voltage signal. The amplitude of the voltage waveform is 0.1-10 V.

One advantage of the two above-described NW-based electroporation methods is the low voltage waveform required to achieve molecular delivery, i.e., under 10 V in amplitude. It is about 100 times lower than those used by commercial electroporation systems. Lowering the required voltage does not only make instrumentation more affordable, it also decreases the likelihood of arcing, which may cause cell death. Further, these methods can be used to deliver molecules to almost all eukaryotic and prokaryotic cells by varying their geometry (e.g., size) or voltage pulses (e.g., amplitude and pulse duration).

Transitioning to prokaryotic cells enables a wealth of new applications including large-scale parallel antibiotic screening. NWs on silicon wafers can be mass produced. This high-throughput, low-cost production allows wide-range application of the delivery methods of this invention.

The details of one or more embodiments are set forth in the accompanying description below. Other aspects, features, and advantages will be apparent from the following drawing, detailed description of embodiments, and also from the appending claims.

### BRIEF DESCRIPTION OF DRAWINGS

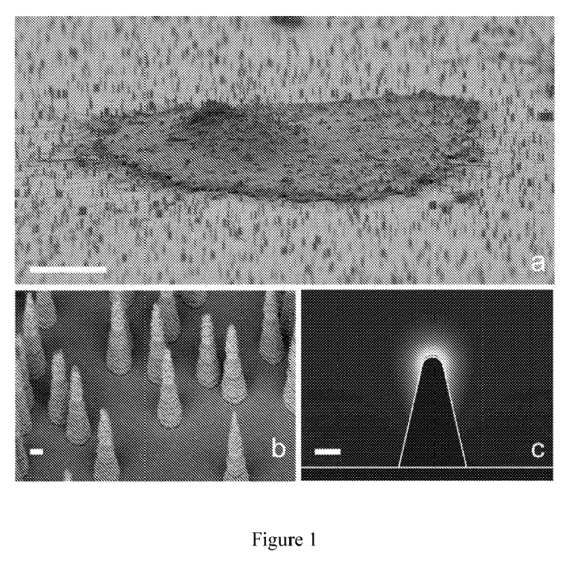

FIG. 1a is a scanning electron microscope (SEM) image of a human fibroblast cultured and fixed atop an array of vertical Si NWs; FIG. 1b is a SEM image of platinum coated Si NWs; and FIG. 1c is a picture showing finite element simulation of electric field enhancement at the tip of an electrically conductive NW. Scale bars: (a) 10 µm, (b) 200 nm, and (c) 200 nm.
FIGs. 2a and b are two fluorescence images showing Hoescht nuclear labeling of HEK293 cells without applying any pulse (control) and with applying a series of voltage pulses having an amplitude of 5.75 V, respectively; FIGs. 2c and d are two fluorescence images showing the absence and presence of a non-membrane-permeable green fluorescent dye (celcein) in HEK293 cells after applying no pulse (control) and a series of voltage pulses having an amplitude of 5.75 V, respectively; FIGs. 2e and f are two fluorescence images showing staining of dead cells after applying no pulse (control) and a series of voltage pulses having an amplitude of 5.75 V, respectively; FIG. 2g is a histogram showing cellular fluorescence intensities; and FIG. 2h is a bar chart showing transfection efficiency and cell viability.

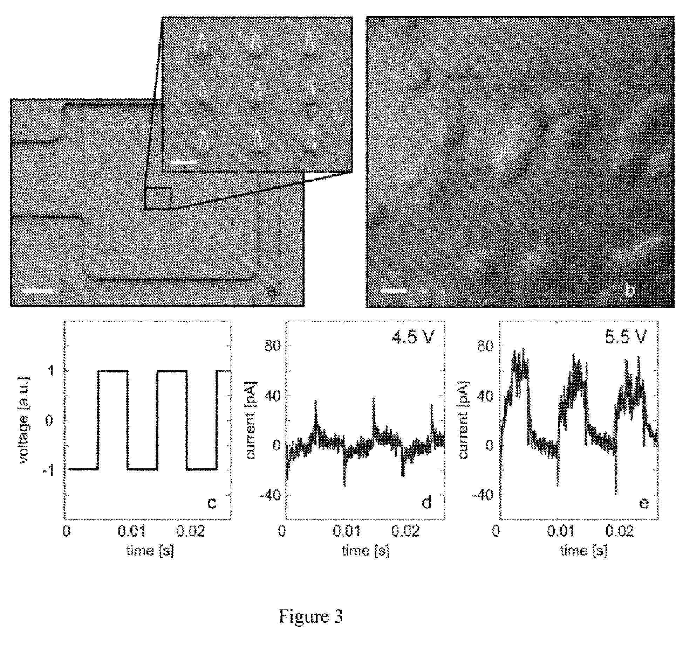

FIG. 3a is a scanning electron image of a set of Pt-tipped NW electrodes fabricated in Silicon on Insulator (The inset is a zoom-in view of the NW electrodes where the termination of the insulating oxide can be seen at the base of the NWs); FIG. 3b is a differential interference contrast image of a HEK293 cell cultured atop a NW electrode. A patch pipette, seen approaching from the lower left, is used to monitor the current injected into the cell via electroporation; FIG. 3c is a diagram showing a voltage waveform applied to the Si NW electrode; and FIGs. 3d and e are diagrams showing amplitudes of current injected into a cell in response to voltage waveforms with amplitudes of 4.5 V and 5.5 V, respectively.

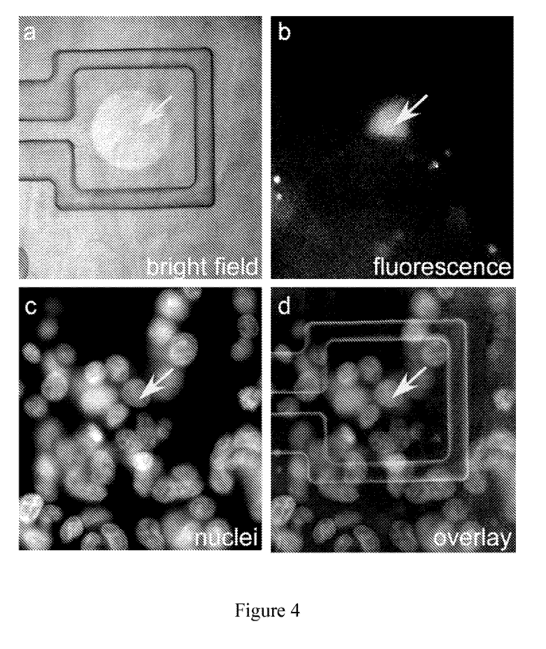

FIG. 4a is a bright field image showing a Si NW electrode with HEK293 cells cultured atop it; FIG. 4b is a fluorescence image of the same area after red fluorescent dye was delivered to a single cell via electroporation; FIG. 4c is a corresponding fluorescence image of the nuclei; and FIG. 4d is a overlay of the above three images. The arrow denotes location of Si NWs.

### DETAILED DESCRIPTION

This invention relates to a NW-based electroporation device and methods of electroporating exogenous molecules into cells.

NWs used in this invention are electrically conductive and attached, preferably in a vertical manner, to a substrate. For *in vitro* electroporation, cells can be cultured directly on such a NW substrate or cultured on another substrate and brought into close contact with the NWs. Alternatively, such a NW substrate can be implanted for *in vivo* or *in situ* biomolecular delivery.

One end of one or more NWs penetrates the basal membrane of a cell and is located inside it. A set of these NWs with their ends inside a cell collectively act as an intracellular or juxtacellular electrode paired with an extracelluar electrode in the bath solution in which the cell was immersed. When an electric potential or current is applied across this pair of electrodes, the intracellular end of each NW can focus the electric field to regions comparable to the radius of curvature at the NW tips (typically < 100 nm in diameter). This nanoscale focusing of the electric field adds an extra degree of freedom to the development of electroporation protocols. The electric field distribution can be controlled by tailoring lithographically the density, aspect ratio, and radius of curvature of the vertical NWs. One can determine the fractional area of the cellular membrane that exceeds the threshold potential, thus enabling efficient and low toxicity electroporation of cells, especially hard-to-transfect-cells, (e.g., stem cells and immune cells). In addition, voltage and current levels, pulse duration, and number of pulses can be optimized to achieve the desired levels of efficiency and viability.

In one embodiment, both NWs and their substrate are electrically conductive. NWs are evenly spread out on the substrate. The NWs can be fabricated on conductive Si wafers in a high-throughput fashion as described in (Shalek, et al., 2010, Proceedings of the National Academy of Sciences, 107, 1870-1875). These NWs can be then coated with metals, which enhance their electric conductivity. Molecular delivery can be achieved by culturing cells atop the NW substrate and applying a current or voltage waveform between the substrate and an electrode in the bath solution. The amplitude required for biomoleular delivery is only a few volts. Almost all of the cells atop the substrate are electroporated and remain viable.

NWs and substrates can be formed of any materials including conductive, semiconductive, and insulating materials, such as silicon, silicon oxide, silicon nitride, silicon carbide, iron oxide, aluminum oxide, iridium oxide, tungsten, stainless steel, silver, platinum, gold, and glass. The electrical conducting layer is formed of a material with low cytoxicity (e.g., gold, silver, and platinum).

In another embodiment, the Si NWs are grown on a substrate as individual sets to allow site-specific delivery of biomolecules into cells. Each individual set is electrically insulated from other sets. Only NWs in the same set are electrically connected and addressable by a voltage waveform independently from other sets. Among a cluster of cells, only one cell is atop an individually-addressable set of vertical NWs and received fluorescent dyes via electroporation. In this way, cell-cell or cell-network interactions can be studied by providing specific perturbations to individual cells within an interacting system.

The insulating layer coated over the NWs is formed of a material with low cytoxicity (e.g., silicon oxide, aluminum oxide, and silicon nitride).

Two approaches are widely used for obtaining an array of NWs on a substrate. One is the so-called bottom-up approach, which essentially involves growing NWs from a precursor material. Taking chemical vapor deposition (CVD) for example, the NW growth process begins by placing or patterning catalyst or seed particles (usually with a diameter of 1 nm to a few hundred nanometers) atop a substrate; next, a precursor material is added to the catalyst or seed particles; and when the particles become saturated with the precursor, NWs begin to grow in a shape that minimizes the device's energy. By varying the precursor, substrate, catalyst/seed particles (e.g., size, density and deposition method on the substrate), and growth conditions, NWs can be made in a variety of materials, sizes, and shapes, at sites of choice. Another approach, the top-down process, essentially involves removing (e.g., by etching) predefined structures from a supporting substrate. For instance, the sites where the NWs are to be formed are first patterned into a soft mask (e.g., photoresist), which is either used to protect the sites that NWs will be formed during a subsequent etch or to pattern a hard mask; an etching step is subsequently performed (either wet or dry) to develop the patterned sites into three-dimensional wires.

Efficiency of Molecular delivery to different cell types can be manipulated by varying the NW size or density.

Contemplated uses of the electroporation methods described above include:
(1) High-throughput biomolecular delivery, in particular, to hard to transfect cells. Applications include transfection, cellular reprogramming, stem-cell differentiation, and probing intra and inter-cellular signaling cascades.
(2) Cell-specific delivery of biomolecules within networks or systems of interacting cells.
(3) Electroporation of adherent cells difficult to resuspend (such as neurons).
(4) Repetitive electroporation at different time points with different doses of the same molecule or various doses of different molecules to the same cells. These cells can remain on the NW substrate between electroporations, since NWs, penetrating the cellular membrane as intracellular electrodes, do not compromise cell viability.

Without further elaboration, it is believed that the above description has adequately enabled the present invention. The following examples are, therefore, to be construed as merely illustrative, and not limitative of the remainder of the disclosure in any way whatsoever.

### Example 1: Fabrication of NW electrode substrates for electroporation

An array of NWs on a silicon substrate was formed by dry-etching a silicon wafer coated with a 200 nm thick thermally-grown silicon oxide layer. To fabricate NWs over large areas, colloidal gold nanoparticles (average diameter 100 nm, purchased from Ted Pella, used after concentrated the purchased sample by about 17 times) were resuspended in a solution of 3% polymethyl-methacrylate (PMMA) in chlorobenzene to form a suspension. The silicon wafer was then spun coated at 3000 RPM with the suspension to produce a 100 nm thick PMMA-nanoparticle film on the wafer's surface. The wafer was then treated with a CF₄ plasma in a reactive ion etching (RIE) device (NEXX DEVICES CIRRUS 150) for 3 minutes to etch the silicon oxide in the regions that were not directly under the gold nanoparticles. The gold nanoparticles were then etched away with a TFA gold etchant to generate a pattern of disconnected silicon oxide dots. The pattern, covering the silicon wafer, acted as a mask for etching the wafer to form vertical NWs. The wafer was etched with an inductively-coupled HBr:O₂ plasma for 10 minutes in another RIE device (SURFACE TECHNOLOGY DEVICES ICP RIE) to form an array of vertically aligned Si NWs (average length: 1000 nm; average diameter: 150 nm; density: 0.5 wire/µm²). The silicon oxide mask was removed by dipping the wafer in 5:1 buffered oxide etchant. The wafer was immediately loaded into an electron beam evaporator where the surfaces of the NWs and the substrate were coated with 100 nm of Pt. Metallic contact to the back side of the wafer was made in a similar fashion.

### Example 2: Plating cells on a NW array

HEK293 cells or fibroblasts between 80-100% confluent were removed from culture flasks by a five minute trypsin treatment. After quenching the enzyme with culture media, the cells were re-suspended to a concentration of 1 million cells/mL. Next, 200 µL of the cell suspension was added to each well of a 48 well cell culture plate containing a silicon substrate with vertically etched NWs prepared in Example 1. The cell culture plate was placed in an incubator (5% CO₂, 90% relative humidity). After 15 minutes of incubation, 150 µL of additional media was added. After 18 hours of additional incubation, the samples were imaged. As shown in FIG. 1a, a human fibroblast cell was attached to the substrate and spread out as a viable cell, despite that it was penetrated by numerous NWs.

### Example 3: Molecular delivery via NW electroporation

Using the method described above, HEK293 cells were plated and cultured atop a silicon substrate with vertically etched NWs prepared in Example 1. NW electrodes were grounded by forming a backside electrical contact to the substrate. A PDMS well surrounding the cell culture was used to confine a solution of phosphate buffered saline (PBS) containing 1 nM of a membrane impermeant dye (calcein). An Ag/AgCl counter electrode was placed into PBS about 0.5 cm above the NW substrate. A biphasic 100 Hz square wave voltage train was applied between the counter electrode and the NW substrate for 0.4 seconds, after 30 seconds the voltage train was repeated. Thirty seconds later the substrate was removed from the dye-loaded PBS and washed through clean PBS and imaged. The amplitude of the voltage pulses was 0 and 5.75 V for the control and delivery experiments, respectively. To assay cell viability, after the electroporation, cells were incubated in EthD-1 for 20 minutes at room temperature and imaged fluorescently. Cells whose membranes had not recovered from the electroporation or were otherwise porous show strong nuclear florescence due to EthD-1 biding to nuclear DNA. These cells were counted as dead, and compared to the total number of cells counted via Hoescht nuclear labeling.

As shown in FIG. 2, this NW electroporation method exhibited a greater than 97% efficiency in delivery of a membrane-impermeable dye (calcein) into HEK293 cells and maintained a more than 85% cell viability. Moreover, cells remained viable atop the NW substrate and could be electroporated repeatedly if necessary.

### Example 4: Fabrication of Individually-addressable NW electrodes

An array of Si NWs on a silicon substrate was formed via several lithography, etching, and deposition steps.

First an etch mask was defined via electron beam lithography (EBL). The silicon on insulator wafer was coated with XR-1541 6% solids negative E-beam resist (Dow Corning) at 2000 RPM to produce a layer of resist approximately 200 nm thick. The wafer was then baked for 2 minutes at 225 °C before electron beam exposure. The Raith-150 EBL tool was used to define 100 nm diameter circles at the locations desired for NW formation. After exposure at a dose of 1000 µC/cm² the wafer was baked again at 225 °C for 4 minutes. The pattern was then developed for 15 seconds in 25% tetramethylammonium hydroxide (TMAH). The resist left behind after developing acted as a hard mask for the subsequent etch process. An inductively-coupled plasma (ICP) of HBr:O₂ was applied for 10 minutes in an ICP-RIE system (SURFACE TECHNOLOGY SYSTEMS) to afford an array of Si NWs (average length: 1000 nm; average diameter: 150 nm; density: 0.5 wire/µm²). The resist mask was then removed by dipping the wafer in 49% hydrofluoric acid. The NWs were then insulated using low pressure chemical vapor deposition (LPCVD) of SiO₂ at 800 °C. To remove the SiO₂ at the NW tips, S1818 photoresist (Microchem) was spun at 3000 RPM and then stripped back using an O₂ plasma (Unaxis RIE) to leave a 500 nm film on the Si substrate. The tips of the NWs which protrude above this layer were then etched (STS ICP-RIE) using a CF₄ plasma to remove the SiO₂ covering the tip. The device was then treated with a 1-min O₂ plasma descum followed by a 10-second dip in buffered oxide etch (BOE) 5:1. The substrate was then loaded into a thermal evaporator where 70nm of evaporated using an electron beam evaporator. The resist was then dissolved for several hours using Remover PG (MicroChem) at 80 °C leaving the metal layer only at the NW tips.

To make the NW electrodes individually addressable, electrode tracts were then patterned by spinning S1818 photoresist (Microchem) on the wafer at 3000 PRM. After baking the wafer for 1 minute at 115 °C, UV contact lithography was used to expose the regions between electrodes. The exposed resist was then developed away using MF-319 (Microchem). The remaining resist served as a mask for ICP-RIE etching (STS) of the Si substrate using a C₄F₈:SF₆ plasma. After stripping the resist with Remover PG, the substrate was coated with 100 nm of Al₂O₃ using atomic layer deposition (ALD) (Cambridge NanoTech). Using contact lithography, 20 micron-diameter areas were exposed around the NWs, as well as 1 x 0.5 mm areas for contact pads. After development, the Al₂O₃ in these regions was removed using TransEtch (Transene). The photoresist was removed and reapplied and the contact regions alone were exposed and developed. After stripping the SiO₂ in these regions using BOE 5:1, a Pt layer was evaporated as before and the photoresist was stripped.

### Example 5: Ionic current injected via electroporation

HEK293 cells were plated and cultured atop a silicon substrate with an individually-addressable set of vertical NWs prepared in Example 4. Transmembrane currents were measured by performing conventional patch clamp measurements in voltage clamp mode while a voltage pulse was applied to the NW electrodes (approximately 0.5 cm away from an Ag/AgCl electrode in the extracellular solution). As shown in FIG.3, an external voltage waveform having an amplitude as low as 5.5 V creates ionic current through a permeabilized cell membrane.

### Example 6: Site-specific biomolecule delivery

HEK293 cells were plated and cultured atop a silicon substrate with an individually-addressable set of vertical NWs prepared in Example 4. Cell specific delivery was achieved following a voltage stimulus similar to FIG. 3 with an amplitude of 4.5 V. The extra cellular solution was PBS containing 1 mg/mL of membrane impermeant fluorescent dye (alexa 647). After the voltage pulse the cells were washed several times through PBS before fluorescence imaging. As shown in FIG. 4, a cell impermeant fluorescent dye was delivered to an individual HEK293 cell atop an individually-addressable set of vertical NWs after the application of a voltage waveform.

Embodiments:
1. A molecular delivery device comprising:
   a substrate having a surface, and
   a plurality of nanowires attached to the surface;
      wherein the substrate is electrically conductive, and the nanowires are coated with an electrically conductive layer.
2. The device of 0, wherein each of the nanowires is attached to the surface along a substantially vertical direction to the surface.
3. The device of 1, wherein each of the nanowires is formed of a semiconductor, a compound semiconductor, a metal oxide, a metal, carbon, boron nitride, or a combination thereof.
4. The device of 3, wherein the semiconductor is silicon.
5. The device of 1, wherein each of the nanowires is 20-10,000 nm in height and 10-500 nm in diameter.
6. The device of 5, wherein the height is 100-5,000 nm and the diameter is 50-250 nm.
7. The device of 6, wherein the height is 800-1,200 nm and the diameter is 70-180 nm.
8. The device of 1, wherein the electrically conductive layer is formed of a metal, a metal oxide, a semiconductor, a compound semicoductor, a metal nitride, or combination thereof.
9. The device of 1, wherein the density of the nanowires is 0.05-10 µm⁻².
10. The device of 9, wherein the density is 0.1-5 µm⁻².
11. The device of 10, wherein the density is 0.2-2 µm⁻².
12. A method of delivering a molecule to a cell, the method comprising:
   providing a substrate having a surface and a plurality of nanowires attached to the surface, wherein both the substrate and nanowires are electrically conductive;
   contacting the nanowires with the cell to allow penetration of one or more nanowires into the cell, wherein the cell is immersed in a bath solution containing the molecule; and
   applying a current or voltage waveform between the substrate and an electrode in the bath solution, whereby the molecule enters into the cell through transiently formed pores on cell membrane.
13. The method of 12, wherein the nanowires are coated with an electrically conductive layer.
14. The method of 12, wherein the molecule is a DNA, a RNA, a protein, a polysaccharide, or a small molecule.
15. The method of 12, wherein the cell is a prokaryotic cell or a eukaryotic cell.
16. The method of 15, wherein the eukaryotic cell is a primary cell, a transformed cell, or a cancerous cell.
17. The method of 16, wherein the primary cell is a neuron, a neuroblast, a beta cell, a myocyte, an osteoblast, a fibroblast, a kerotinocyte, a monocyte, an immune cell, a stem cell, or an oocyte.
18. The method of 12, wherein the amplitude of the voltage waveform is 0.1-10 V.
19. The method of 18, wherein the amplitude is 3-7 V.
20. The method of 19, wherein the amplitude is 4-6 V.
21. A method of delivering a molecule to a cell, the method comprising:
   providing a substrate having a surface and a plurality of electrically conductive nanowires, each of which has a first end and a second end, wherein the substrate and each of the nanowires are coated with an electrically insulating layer except for the first and second ends of each of the nanowires, the first end is attached to the surface, and the second end is coated with an electrically conductive layer;
   contacting the nanowires with the cell immersed in a bath solution containing the molecule to allow penetration of one or more nanowires into the cell; and
   applying a current or voltage waveform between two electrodes, one connected to the first end of each of the nanowires and the other placed in the bath solution, whereby the molecule enters into the cell through transiently formed pores on cell membrane.
22. The method of 21, wherein the molecule is a DNA, a RNA, a protein, a polysaccharide, or a small molecule.
23. The method of 21, wherein the cell is a prokaryotic cell or a eukaryotic cell.
24. The method of 23, wherein the eukaryotic cell is a primary cell, a transformed cell, or a cancerous cell.
25. The method of 24, wherein the primary cell is a neuron, a neuroblast, a beta cell, a myocyte, an osteoblast, a fibroblast, a kerotinocyte, a monocyte, an immune cell, a stem cell, or an oocyte.
26. The method of 21, wherein the amplitude of the voltage waveform is 0.1-10 V.
27. The method of 26, wherein the amplitude is 3-7 V.
28. The method of 27, wherein the amplitude is 4-6 V.

## Claims

1. A molecular delivery device comprising:
a substrate having a surface; and
a plurality of nanowires attached to the surface;
wherein the substrate is electrically conductive and the nanowires are coated with an electrically conductive layer formed of a metal oxide.

2. The device of claim 1, wherein the metal oxide is selected from iridium oxide, indium tin oxide and zinc oxide.

3. The device of claim 1 or claim 2, wherein the nanowires are formed of a semiconductive or insulating material.

4. The device of claim 3, wherein the semiconductive or insulating material is selected from silicon, silicon oxide, silicon nitride, silicon carbide, iron oxide and aluminum oxide.

5. The device of claim 1 or claim 2, wherein the nanowires are formed of a semiconductor, a compound semiconductor, a metal oxide, a metal, carbon, boron nitride, or a combination thereof.

6. The device of claim 5, wherein the semiconductor is silicon.

7. The device of any preceding claim, wherein each of the nanowires is attached to the surface of the substrate along a substantially vertical direction to the surface.

8. The device of any preceding claim, wherein the nanowires have a height of 20-10,000 nm and a diameter of 10-500 nm.

9. The device of claim 8, wherein the nanowires have a height of 100-5,000 nm and a diameter of 50-250 nm.

10. The device of claim 9, wherein the nanowires have a height of 800-1,200 nm and a diameter of 70-180 nm.

11. The device of any preceding claim, wherein the density of the nanowires is 0.05-10 nanowires per µm².

12. The device of claim 11, wherein the density of the nanowires is 0.1-5 nanowires per µm².

13. The device of claim 12, wherein the density of the nanowires is 0.2-2 nanowires per µm².
